# EUROPEAN PATENT APPLICATION

(11) **EP 3 090 682 A1**
(43) Date of publication of application: **09.11.2016**
(21) Application number: 15166962.9
(22) Date of filing: 08.05.2015
(51) Int. Cl.: A61B 5/00, A61B 8/08

(54) **ARTIFACT REDUCTION IN PHOTOACOUSTIC AND THERMOACOUSTIC IMAGING**

(71) Applicant: UNIVERSITEIT TWENTE, 7522 NB Enschede (NL)
(72) Inventor: Steenbergen, Wiendelt, 7522 NB Enschede (NL); Kuniyil Ajith Singh, Mithun, 7522 NB Enschede (NL)
(74) Representative: Nederlandsch Octrooibureau

(57) **Abstract**

A method of reflection artifact reduction in photoacoustic or thermoacoustic imaging comprises generating an object image of an object by causing a radiation emitting device to emit an electromagnetic radiation and causing an acoustic receiver to detect an acoustic response. A high intensity region is determined in the object image, said high intensity region corresponding to a radiation absorbing region within the object. An artifact detection image is generated by causing an acoustic transmitter to emit a focused ultrasound beam based on a location of the radiation absorbing region and causing an acoustic receiver to detect an acoustic reflected signal. The object image is corrected based on the artifact detection image.

## Description

### FIELD OF THE INVENTION

The invention relates to photoacoustic imaging. The invention further relates to thermoacoustic imaging. Moreover, the invention relates to artifact reduction. More particularly, the invention relates to artifact reduction in photoacoustic or thermoacoustic imaging.

### BACKGROUND OF THE INVENTION

Photoacoustic (PA) imaging is an imaging modality that can be used for biomedical applications. Related imaging technology includes thermoacoustic imaging. Photoacoustic imaging is typically performed using pulsed visible or near-infrared light. This light, while scattering through soft tissue, is absorbed at specific locations by absorber molecules such as hemoglobin in blood. The absorbed energy is converted into heat; the subsequent thermoelastic expansion causes ultrasound (US) to be produced from the absorbing region. The US is measured at the surface of tissue using US detectors and the acquired signals are used to reconstruct the location and spatial details of the absorber. PA imaging combines the advantages of optical and US imaging, providing optical spectroscopic contrast with ultrasonic resolution. While US imaging utilizes acoustic impedance mismatches in tissue for its signals to provide structural details, PA imaging extracts functional information based on optical absorption by e.g. blood. Related imaging technology includes thermoacoustic imaging, in which any kind of e.g. electromagnetic radiation can be used instead of light to generate the thermoacoustic effect. However, some undesired imaging artifacts may be present in the images.

WO 2014/01660 A1 discloses generating patterns of vibration within the body to produce localized displacements at a region and at clutter-producing sites in order to eliminate the clutter. WO 2014/076674 discloses medical image acquiring device configured for repeatedly photoacoustically imaging an intracorporeal spatial location that is common image to image, varying at least one of ultrasound transducer acquisition location, and light excitation, image to image so that averaging of the images serves to decorrelate background clutter that exists in the images, of said spatial location, individually. EP2638851 discloses a subject information obtaining device comprising a light intensity distribution obtaining means arranged to obtain a first light intensity distribution within the subject when irradiating light in the first measurement state on a subject, and a second light intensity distribution within the subject when irradiating light in the second measurement state on the subject, and display means so as to compare the first image data and the second image data at the display means.

However, it would be advantageous to improve the image quality of PA imaging and/or thermoacoustic imaging.

### SUMMARY OF THE INVENTION

It would be advantageous to provide an improved photoacoustic or thermoacoustic imaging method. To address this concern, in a first aspect of the invention, a method of reflection artifact reduction in photoacoustic or thermoacoustic imaging by an imaging apparatus is provided, the method comprising
generating an object image of an object by causing a radiation emitting device to emit an electromagnetic radiation and causing an acoustic receiver to detect an acoustic response;
determining a high intensity region in the object image, said high intensity region corresponding to a radiation absorbing region within the object;
generating an artifact detection image by causing an acoustic transmitter to emit a focused ultrasound beam based on a location of the radiation absorbing region and causing an acoustic receiver to detect an acoustic reflected signal; and
correcting the object image based on the artifact detection image.

The radiation absorbing region may coincide with a radiation absorbing substance, i.e. a substance that absorbs the electromagnetic radiation and generates acoustic signals in the process of absorbing the radiation. The acoustic signal generated by the radiation absorbing region may be emitted from the radiation absorbing region in several different directions, or may be undirected. Some of the acoustic rays emitted from the radiation absorbing region will travel directly to the acoustic detector and be detected. Some of the acoustic rays that may travel in other directions may never reach the detector. However, some of the acoustic rays may be reflected by other structures having different acoustic impedance from their surroundings. A part of these indirect acoustic rays may reach the detector and be detected. Such detected indirect acoustic rays, without being recognized as such by the detector, may be interpreted as direct rays from an absorber material, and thus lead to artifacts in the photoacoustic or thermoacoustic image due to different angle of incidence to the detector and/or arrival time at the detector compared to the direct ray.

The artifact detection image generated by emitting a focused ultrasound beam based on a location of the radiation absorbing region corresponding to said high intensity region and detecting an acoustic reflected signal, helps to discover, for example, the acoustic signals that cause these artifacts.

The focused ultrasound beam may be focused on said radiation absorbing region corresponding to said high intensity region. This way, the focused ultrasound beam is focused on the region where e.g. the photoacoustic or thermoacoustic substance is located. The ultrasound beam then in a way mimics the ultrasound signals emitted from the photoacoustic or thermoacoustic substance corresponding to the high-intensity region. Thus, the reflections caused by those ultrasound signals can be detected.

The focused ultrasound beam may be emitted and the acoustic reflected signal may be detected while refraining from emitting the electromagnetic radiation. This helps to distinguish reflection artifacts from absorbers of electromagnetic radiation.

The method may comprise processing the detected acoustic reflected signal based on a position of the at least one high intensity region. The detected acoustic reflected signal may be processed taking into account the position of the high intensity region in the object image. This position corresponds to the position of the absorbing region. By taking this position into account, the acoustic signals may be corrected for the difference in locations between the ultrasound transmitter and the sound source constituted by the radiation absorbing region.

The method may comprise mapping a position in the artifact detection image to a position in the object image based on a position of the high intensity region. This allows the correction to be based on the mapped positions.

The mapping may be based on a time of flight of the focused ultrasound beam from the acoustic transmitter to the radiation absorbing region corresponding to said at least one high intensity region. This time of flight is important for the mapping because the focused ultrasound beam has a longer time of flight compared to acoustic reflections of the sound signal generated by the radiation absorbing region, because the latter signal starts at the absorbing region, whereas the focused ultrasound beam first has to travel from the ultrasound transmitter to the absorbing region.

The mapping may comprise subtracting said time of flight from a detection time of the detected acoustic reflected signal. This way the difference in time of flight of the received signals is taken into account in a way that helps to localize the artifacts.

The method may comprise determining said time of flight based on a distance from the acoustic transmitter to the radiation absorbing region corresponding to said at least one high intensity region, and an estimate of a speed of sound of the emitted focused ultrasound beam. This way, the time of flight may be computed based on the begin location, end location, and the speed.

The method may comprise determining said time of flight based on a detection time of the detected acoustic response to the electromagnetic radiation, said acoustic response corresponding to the high intensity region. The detection time of the detected acoustic response corresponding to the high intensity region when generating the object image is a way to measure said time of flight.

The method may comprise
determining a plurality of high intensity regions in the object image, said high intensity regions corresponding to respective radiation absorbing regions within the object;
generating a plurality of artifact detection images by causing the acoustic transmitter to emit a sequence of focused ultrasound beams being focused on the respective radiation absorbing regions corresponding to different ones of the high intensity regions in the object image, and causing the acoustic receiver to detect an acoustic reflected signal generated by each of the focused ultrasound beams; and
correcting the object image based on the plurality of artifact detection images.

This allows to detect reflection artifacts generated by a plurality of photoacoustic or thermoacoustic regions.

The method may comprise causing the acoustic transmitter to focus the ultrasound beams on radiation absorbing regions corresponding to high intensity regions in the object image for which there is another high intensity region in the object image further away from the acoustic detector. This can save time (acquisition time and/or processing time) as the high intensity regions furthest away from the acoustic detector may likely be artifacts that do not correspond to any radiation absorbing region.

The method may comprise generating a plurality of artifact detection images by causing the acoustic transmitter to emit a sequence of focused ultrasound beams based on the same radiation absorbing region corresponding to the high intensity region, wherein the sequence of focused ultrasound beams have different inclination angles with respect to the object, and causing the acoustic receiver to detect a respective acoustic reflected signal generated by each of the focused ultrasound beams; and correcting the object image based on the plurality of artifact detection images. This may allow to further refine the artifact detection by simulating the acoustic waves emitted by the radiation absorbing region in different regions.

In another aspect of the present disclosure, a system for artifact reduction in photoacoustic or thermoacoustic imaging is provided. The system comprises
a first imaging unit for generating an object image of an object by causing a radiation emitting device to emit an electromagnetic radiation and causing an acoustic receiver to detect an acoustic response;
an image analyzing unit for determining a high intensity region in the object image, said high intensity region corresponding to a radiation absorbing region within the object;
a second imaging unit for generating an artifact detection image by causing an acoustic transmitter to emit a focused ultrasound beam based on a location of the radiation absorbing region and causing an acoustic receiver to detect an acoustic reflected signal; and
an image correction unit for correcting the object image based on the artifact detection image.

The system may further comprise the radiation emitting device for emitting the electromagnetic radiation; the acoustic transmitter for emitting the focused ultrasound beam; and the acoustic receiver or receivers for detecting the acoustic response and the acoustic reflected signal.

According to another aspect of the present disclosure, a computer program product is provided, possibly stored on a non-transitory media, comprising instructions causing a processor to perform the steps of:
generating an object image of an object by causing a radiation emitting device to emit an electromagnetic radiation and causing an acoustic receiver to detect an acoustic response;
determining a high intensity region in the object image, said high intensity region corresponding to a radiation absorbing region within the object;
generating an artifact detection image by causing an acoustic transmitter to emit a focused ultrasound beam based on a location of the radiation absorbing region and causing an acoustic receiver to detect an acoustic reflected signal; and
correcting the object image based on the artifact detection image.

The person skilled in the art will understand that the features described above may be combined in any way deemed useful. Moreover, modifications and variations described in respect of the method may likewise be applied to the system and to the computer program product, and modifications and variations described in respect of the system may likewise be applied to the method and to the computer program product.

### BRIEF DESCRIPTION OF THE DRAWINGS

In the following, aspects of the invention will be elucidated by means of examples, with reference to the drawings. The drawings are diagrammatic and are not drawn to scale. In the drawings, similar objects are denoted by the same reference numerals.
Fig. 1A is an illustration of photoacoustic signal generation and signal causing reflection artifacts.
Fig. 1B is an illustration of acoustic imaging by focusing ultrasound onto the photoacoustic source and detecting the resulting reflection.
Fig. 2A is an illustration of an object to be imaged.
Fig. 2B is an illustration of a photoacoustic image.
Fig. 2C is an illustration of a focused ultrasound transmission.
Fig. 2D is an illustration of another focused ultrasound transmission.
Fig. 2E is an illustration of an image revealing reflection artifacts.
Fig. 2F is an illustration of a corrected photoacoustic image.
Fig. 3 is a block diagram of an imaging apparatus.
Fig. 4 is a flowchart of a method of artifact reduction in photoacoustic imaging.

### DETAILED DESCRIPTION OF EMBODIMENTS

In the following description, a number of example embodiments will be described in more detail. However, the description of these embodiments is not intended to limit the scope of protection. Also, examples of photoacoustic imaging will be described in the following. However, it is noted that in view of the present disclosure, the techniques described in respect to photoacoustic imaging may be applied in a similar way to any imaging methods in which electromagnetic radiation is converted to ultrasound through absorption of the radiation in the radiated tissue, such as thermoacoustic imaging.

Dual mode ultrasound (US) and photoacoustic (PA) probes typically use reflection mode imaging geometry, where light irradiation is done from the same side at which PA signals are detected. The influence of acoustic reflectors and their reflection artifacts is an important problem in reflection mode photoacoustic imaging. However, these acoustic reflectors and their reflection artifacts may also be apparent in other acquisition modes such as transmission mode photoacoustic imaging. Absorption of light by skin and superficial optical absorbers may produce photoacoustic transients, which penetrate into the tissue and get reflected from structures having different acoustic impedance. These reflected photoacoustic signals, when reconstructed may appear in the region of interest, which causes difficulties in interpreting the images. Furthermore, when imaging structures such as finger joints (multiple light absorbers and acoustic reflectors), signals of interest may become mixed with the reflections, which result in the incorrect interpretation of images. Considering the light scattering and acoustic attenuation in tissue, reflected PA signals from a particular depth are higher in amplitude than the signals of interest generated at that depth, which significantly reduces the image contrast. Since these artifacts are triggered by the properties of the tissue, simple signal averaging is not effective for reducing it. Thus, it is desirable to develop methods for eliminating (or at least reducing) reflection artifacts to achieve clinically relevant PA imaging depth.

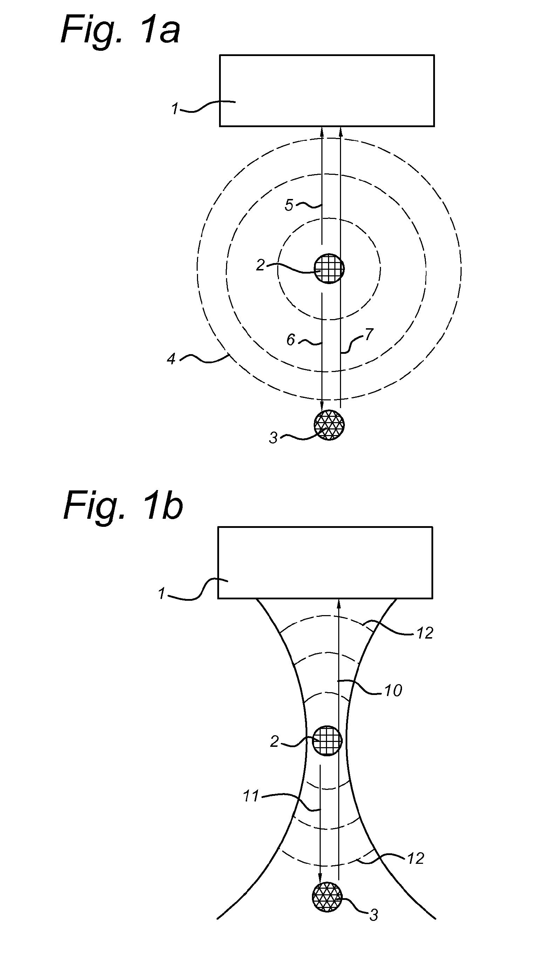

PhotoAcoustic-guided Focused UltraSound imaging (PAFUSion), as its name suggests is a method combining US and PA imaging to eliminate reflection artifacts in PA images. This technique ultrasonically simulates the PA waves from the optical absorber, traversing towards the acoustic reflector and then to the detector, and thus offers an effective way to mimic the PA reflection signal. Fig. 1A shows the generation of a PA signal from an optical absorber 2 and the PA reflection signal caused by the acoustic reflector 3 underneath it. When being exposed to light radiation, the optical absorber 2 emits ultrasound waves in all directions. The wave front is illustrated by dashed circles 4. A direct ultrasound wave 5 reaches the ultrasound detector 1 directly. Another ultrasound wave 6 reaches an acoustic reflector 3 and reflected acoustic wave 7 reaches the ultrasound probe 1 indirectly. Thus, acoustic reflector 3 and reflected acoustic wave 7 may cause an undesired artifact in a photoacoustic image, because it is difficult to distinguish direct photoacoustic ultrasound waves from reflected photoacoustic ultrasound waves. The light emitter has not been drawn in the figure, but it may be combined with the ultrasound probe 1. Ultrasound probe 1 may be touching the skin surface (not illustrated), and the optical absorber 2 and acoustic reflector 3 may be objects within the body of a patient, for example.

Fig. 1B illustrates an image acquisition to help detect and/or reduce the reflection artifacts. In Fig. 1B, the ultrasound probe 1, the optical absorber 2, and the acoustic reflector 3 are the same as those of Fig. 1A. However, no light waves are emitted and therefore no photoacoustic ultrasound signal is generated by the optical absorber 2. Instead, an ultrasound signal is emitted, for example from the ultrasound probe 1, into the body. As illustrated by wavefronts 12, the ultrasound is focused on the optical absorber 2. The location of the optical absorber may have been determined beforehand by the guidance of PA data. The ultrasound waves 12, e.g. wave 11, from the ultrasound probe 1 that have passed the optical absorber 2 thus mimic the ultrasound waves 4 traversing towards the reflector 3 when the optical absorber 2 is activated optically as shown in Fig. 1A. As a result, US reflections 10 of the ultrasound waves 12 from the acoustic reflector 3 are correlated with the PA signal reflection in terms of shape and depth.

For example, the US reflections from the acoustic reflector 3 will be similar to the PA signal reflection in terms of shape and depth, if the reconstruction is done without considering two way propagation of sound. The time at which US reaches the focus distance is set as the start time of acquisition (t =0) while performing the reconstruction (reconstruction is performed considering the structure at focus depth as PA source). Thus PA signal reflections can be reconstructed using this technique, which then can be used to correct PA images for reflection artifacts.

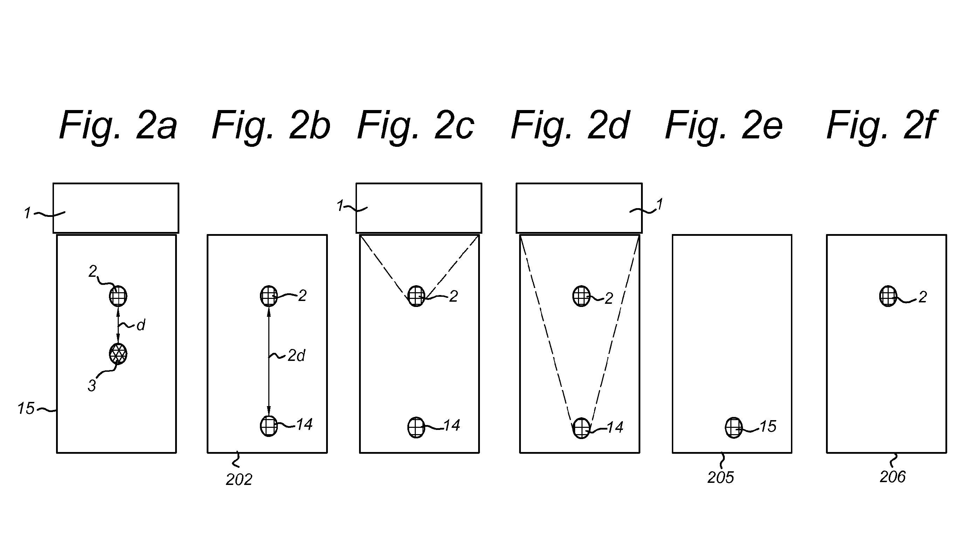

Fig. 2 illustrates several aspects of the image acquisition and image processing techniques. Fig. 2A illustrates the setup of the ultrasound probe 1 on a body 15 comprising an optical absorber 2 and an acoustic reflector 3, separated by a distance d. Fig. 2B illustrates a possible PA image 202 of the PA acquisition in the setup of Fig. 2A. Because PA image reconstruction assumes that the received acoustic signals are directly emitted from an optical absorber, increased times of flight of received signals due to acoustic reflections results in reconstruction of objects further away from the ultrasound probe 1. Thus, the reflection artifact caused by acoustic reflector 3 is reconstructed as artifact 14 at a greater distance of, in this case, 2d from the actual optic absorber 2.

Fig. 2C illustrates a focused ultrasound beam in which the generated ultrasound is focused on the detected optical absorber 2.

Fig. 2E illustrates an artifact detection image 205. This image 205 is a specially treated US image 205 based on the detected acoustic reflections of the focused ultrasound beam shown in Fig. 2C. The special treatment may involve processing the detected acoustic reflections as if they were acoustic signals obtained from photoacoustic imaging. Herein, the start time of the acquisition may be set to be equal to the arrival time of the focused ultrasound wave at the optical absorber 2. That is, the arrival time of the detected acoustic signals may be reduced by the time of flight from the acoustic transmitter to the optical absorber 2.

The acoustic reflector 3 reflects the focused ultrasound signal and is reconstructed as a high intensity region 15 at the position corresponding to the reflection artifact 14 of the photoacoustic image 202. By correcting the photoacoustic image 202 based on the artifact detection image 205, the artifact 14 corresponding to high intensity region 15 can be eliminated, resulting in artifact-free image illustrated in Fig. 2F. The correction can be done, for example, by subtracting the artifact detection image from the photoacoustic image, for example using a weighted subtraction. Other ways of correcting the photoacoustic image based on the artifact detection image are also possible, for example by detecting the high intensity region 15 in the focused ultrasound image and reducing the intensity of the corresponding artifact 14 in the photoacoustic image.

High intensity points in the PA image 202 are first identified and the technique of generating the artifact detection image may be applied to each high intensity point individually, preferably from the top (close to the US probe) to the bottom (further away from the US probe). The different foci of the ultrasound wave are illustrated in Fig. 2C and Fig. 2D. During each step, US reflections from the depth of focus and above it (closer to the US probe) may be ignored.

The artifact detection images may be combined by means of weighted addition or in another way. When multiple focused ultrasound acquisitions are performed, they may be added using weighted addition, wherein each weight may depend on the pixel intensity of the PA image at the focus depth of the relevant image. The weighted addition of the images obtained at each step yields an image in which the PA acoustic reflection artifacts are exposed, as in Fig. 2E. Furthermore, this data can be used to treat the PA image to obtain a reflection artifact-free PA image 206, as illustrated in Fig. 2F.

In a variation on this procedure, the steps visualized in Fig. 2C and Fig. 2D can be performed with the US beam injected at various angles, keeping the focal spot of the ultrasound beam coincide with the high intensity region of the PA image. This will enable to identify reflections on reflecting objects which are laterally displaced with respect to the absorbing structure. However, some laterally displaced reflections may also be detected without injecting the US beam at various angles.

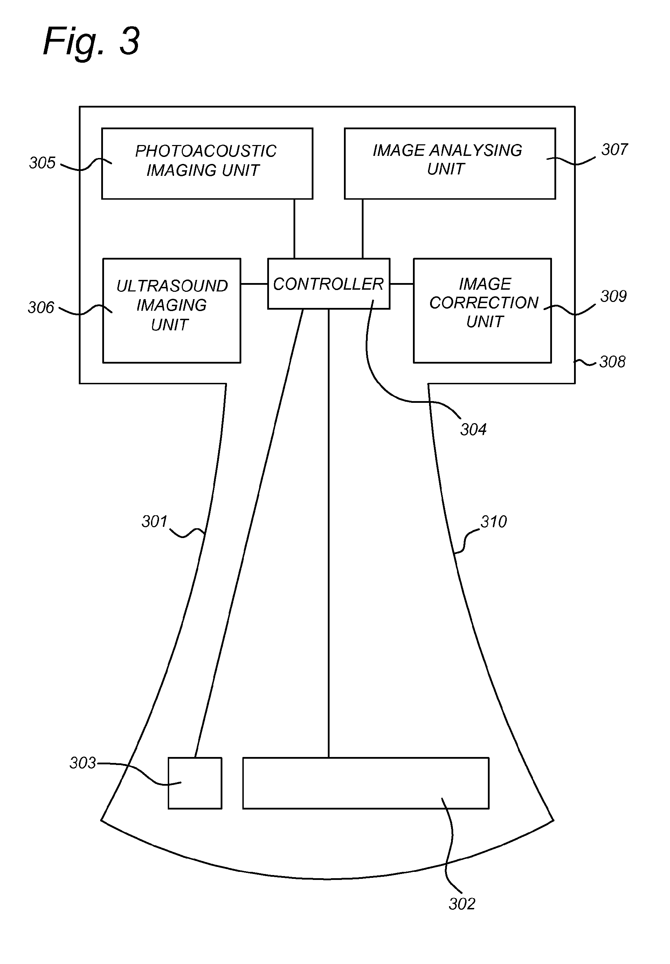

Fig. 3 illustrates a photoacoustic imaging system, with the capability to reduce artifacts. As illustrated the system comprises a control and signal processing section 308 and a sensor section 310. These sections may be implemented by means of separate devices, or may be integrated into a single device. The sensor section 310 comprises an ultrasound transmitter/receiver 302 and an electromagnetic radiation emitting device 303. It will be understood that the ultrasound transmitter/receiver 302 may be divided into a separate ultrasound transmitter and ultrasound receiver, although this is not illustrated in the drawing. For example, in an alternative embodiment, separate probes maybe used for transmission and reception. The drawing illustrates a combined ultrasound transmitter/receiver 302, i.e. an ultrasound transceiver, as an example. Moreover, it will be understood that many different variants of the electromagnetic radiation emitting device 303 are possible. For example, the electromagnetic radiation emitting device 303 may be a light generator such as a light source or lamp, for example luminescent emitting diode (LED), laser, or any other kind of light source, electromagnetic radiation source, or proton beam source. Other examples of usable electromagnetic radiation emitting devices include microwave generator and radio, e.g. radio frequency (RF) pulse generator. To build the sensor section 310, components known in the art of photoacoustic imaging may be used as the electromagnetic radiation emitting device 303 and the acoustic transmitter/receiver 302. For example, the acoustic transmitter/receiver 302 is an acoustic transceiver 302 comprising an array of acoustic transceiver elements that can cooperate to generate a directed and/or focused acoustic signal and/or to determine the direction and/or origin of any incoming acoustic signals.

The control and signal processing section 308 may comprise a controller 304. For example, the controller 304 is a central processing unit of a computer. Storage means or memory (not shown) are provided for storing the images. Said storage means or memory may also store computer program code. The photoacoustic imaging unit 305, ultrasound imaging unit 306, image analyzing unit 307, and image correction unit 309 may be implemented by means of computer program code stored in the memory. Such program code may cause the processor to perform actions such as processing data and controlling the electromagnetic radiation emitting device 303 and the acoustic transceiver 302. Another way to implement these units is by means of a dedicated electronic circuitry.

The photoacoustic imaging unit 305 is configured to generate a photoacoustic image of an object by causing a radiation emitting device to emit an electromagnetic radiation and causing an acoustic receiver to detect an acoustic response. The electromagnetic radiation triggers a thermo-elastic effect when the electromagnetic radiation is absorbed by an absorber. The thermo-elastic effect involves generation of sound.

The detected acoustic response signals are processed by the photoacoustic imaging unit 305 to generate the photoacoustic image. To this end, photoacoustic imaging algorithms may be used, which are known in the art per se. These algorithms use data from an array of acoustic detector elements to localize a sound source of a detected acoustic signal.

The image analyzing unit 307 is configured to determine at least one high-intensity region in the photoacoustic image. Herein, a high-intensity region is a region from where a sound originated according to the photoacoustic imaging algorithm. The found high-intensity region is mapped to a location in space in relation to the sound detector.

The ultrasound imaging unit 306 is configured to generate an artifact detection image, without emitting the electromagnetic radiation (e.g. light). To that end, the acoustic transceiver 302 is controlled to emit an ultrasound signal, and to detect any acoustic reflected signals. More particularly, the acoustic transceiver 302 may be configured to generate a focused ultrasound beam. The focal region of the focused ultrasound beam may be controlled to coincide with a region of the object corresponding to said intensity region which was determined as a source of the received acoustic signal. Further, the acoustic transceiver 302 may be controlled to detect an acoustic reflected signal. The ultrasound imaging unit 306 processes the received data signals to localize acoustic reflectors. Specific ways to localize the acoustic reflectors and map them to the corresponding locations in the photoacoustic image are described elsewhere in this disclosure.

The image correction unit 309 is configured to correct the photoacoustic image based on the artifact detection image. Specific ways to perform this correction are described elsewhere in this disclosure. A simple example is subtracting the artifact detection image from the photoacoustic image.

Although in the example of Fig. 3, a photoacoustic imaging unit 305 is shown, this may be replaced by a thermoacoustic imaging unit or another imaging unit mutatis mutandis, depending on the radiation emitted by the electromagnetic radiation emitting device 303.

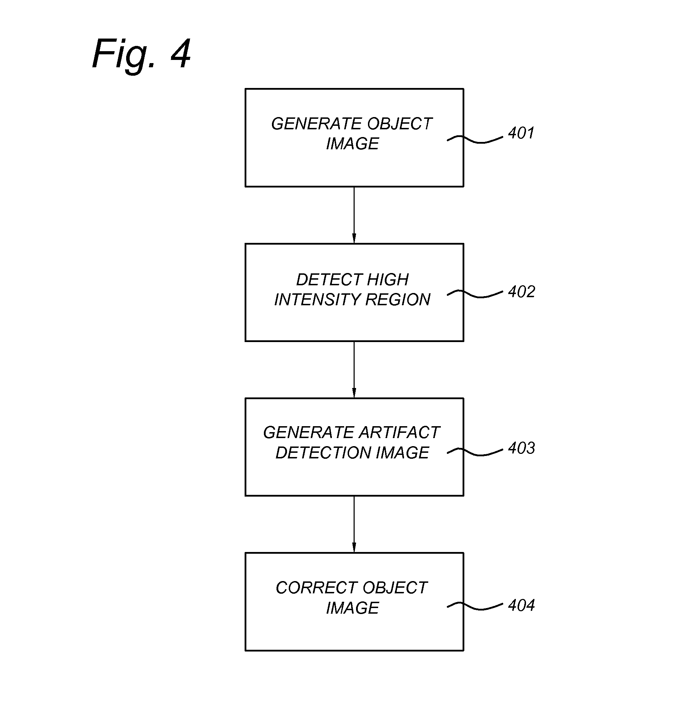

Fig. 4 illustrates a method of reflection artifact reduction in photoacoustic imaging. The method comprises, in step 401, generating a photoacoustic image of an object by causing the radiation emitting device to emit an electromagnetic radiation and causing the acoustic receiver to detect an acoustic response. Next, in step 402, at least one high intensity region is detected in the photoacoustic image. The high intensity region is a region which corresponds to a source of detected acoustic response. This may be a real photoacoustic source or e.g. a reflection artifact. In step 403, an artifact detection image is generated by causing the acoustic transmitter to emit a focused ultrasound beam based on a region corresponding to said at least one high intensity region and causing an acoustic receiver to detect an acoustic reflected signal. For example, the focused ultrasound beam is focused on said region corresponding to said at least one high intensity region. While emitting the ultrasound beam and detecting the acoustic reflected signal, the electromagnetic radiation emitting device is controlled not to emit radiation.

The detected acoustic reflected signal may be processed based on a position of the at least one high acoustic intensity region in the photoacoustic image. For example, the detection time of the acoustic reflected signal is adjusted based on a time of flight from the acoustic transmitter to the focal region of the ultrasound beam or to the high intensity region.

In step 404, the photoacoustic image is corrected based on the artifact detection image. This correction may involve removing reflection artifacts from the photoacoustic image by comparing the photoacoustic image with the artifact detection image. The correction may be based on a mapping of a position in the artifact detection image to a position in the photoacoustic image. This mapping may be based on a position of the high acoustic intensity region. Due to the different path followed by the acoustic wave generated by the photoacoustic source and the acoustic wave generated by the acoustic transmitter, the locations of high intensity regions in the acoustic image and corresponding high intensity regions caused by artifacts in the photoacoustic image may differ. The mapping maps these corresponding regions to each other. The mapping may be based on a time of flight of the focused ultrasound beam from an acoustic transmitter to the region corresponding to said at least one high intensity region. For example, to generate the mapping, said time of flight may be subtracted from a detection time of the detected acoustic reflected signal. The time of flight itself may be computed, for example, based on a position of an acoustic transmitter, a position of the region corresponding to said at least one high intensity region, and an estimate of a speed of sound of the emitted focused ultrasound beam. Alternatively, the time of flight may be determined based on a detection time of the detected photoacoustic response corresponding to the high intensity region.

It is possible to generate a plurality of artifact detection images in step 403. These artifact detection images are individually made in the same way described above. However, some parameters may be varied in between these acquisitions. For example, different high intensity regions may be treated by focusing the ultrasound beam on different focal regions in the different acquisitions. Further, for a single focal region, the inclination angle may be varied to collect reflections from more acoustic reflectors, thus being able to eliminate more artifacts.

For example, in step 402, a plurality of high intensity regions may be detected in the photoacoustic image, for example by a threshold technique or another well known image processing technique. And in step 403, a plurality of artifact detection images may be generated by causing the acoustic transmitter to emit a sequence of focused ultrasound beams being focused on respective regions corresponding to different ones of the at least one of the high intensity regions in the photoacoustic image, and causing the acoustic receiver to detect an acoustic reflected signal generated by each of the focused ultrasound beams. Thus, each artifact detection image may have a different high intensity region associated therewith. However, this is not a limitation. An alternative is that there are a plurality of artifact detection images (or at least one artifact detection image) for each high intensity region. In step 404, the photoacoustic image may be corrected based on the plurality of artifact detection images.

In steps 402 and/or 403, a selection may be made regarding the high intensity regions for which an artifact detection image will be generated. For example, only those high intensity regions may be selected for which there is another high intensity region further away from the detector. The acoustic transmitter thus focuses the ultrasound beams on those regions corresponding to high intensity regions in the photoacoustic image for which there is another high intensity region in the photoacoustic image further away from the acoustic detector. The regions furthest away are more likely to be artifacts themselves, and it is not necessary to obtain the corresponding artifact detection image for correction.

As mentioned before, in step 403 it is possible to generate a plurality of artifact detection images by causing the acoustic transmitter to emit a sequence of focused ultrasound beams based on said region corresponding to said at least one high intensity region, wherein the sequence of focused ultrasound beams have different inclination angles with respect to the object, and causing the acoustic receiver to detect a respective acoustic reflected signal generated by each of the focused ultrasound beams. In step 404, the photoacoustic image may be corrected based on the plurality of artifact detection images.

Some or all aspects of the invention may be suitable for being implemented in form of software, in particular a computer program product. Such computer program product may comprise a storage media, such as a memory, on which the software is stored. Also, the computer program may be represented by a signal, such as an optic signal or an electro-magnetic signal, carried by a transmission medium such as an optic fiber cable or the air. The computer program may partly or entirely have the form of source code, object code, or pseudo code, suitable for being executed by a computer system. For example, the code may be executable by one or more processors.

The examples and embodiments described herein serve to illustrate rather than limit the invention. The person skilled in the art will be able to design alternative embodiments without departing from the scope of the claims. Reference signs placed in parentheses in the claims shall not be interpreted to limit the scope of the claims. Items described as separate entities in the claims or the description may be implemented as a single hardware or software item combining the features of the items described.

## Claims

**1.** A method of reflection artifact reduction in photoacoustic or thermoacoustic imaging by an imaging apparatus, comprising
generating an object image of an object by causing a radiation emitting device to emit an electromagnetic radiation and causing an acoustic receiver to detect an acoustic response;
determining a high intensity region in the object image, said high intensity region corresponding to a radiation absorbing region within the object;
generating an artifact detection image by causing an acoustic transmitter to emit a focused ultrasound beam based on a location of the radiation absorbing region and causing an acoustic receiver to detect an acoustic reflected signal; and
correcting the object image based on the artifact detection image.

**2.** The method of claim 1, comprising causing the focused ultrasound beam to be focused on said radiation absorbing region.

**3.** The method of claim 1, comprising causing the focused ultrasound beam to be emitted and the acoustic reflected signal to be detected while controlling the radiation emitting device to refrain from emitting the electromagnetic radiation.

**4.** The method of claim 1, comprising processing the detected acoustic reflected signal based on a position of the at least one high intensity region in the object image.

**5.** The method of claim 1, comprising mapping a position in the artifact detection image to a position in the object image based on a position of the high intensity region in the object image.

**6.** The method of claim 5, wherein the mapping is based on a time of flight of the focused ultrasound beam from the acoustic transmitter to the radiation absorbing region corresponding to said at least one high intensity region.

**7.** The method of claim 6, wherein the mapping comprises subtracting said time of flight from a detection time of the detected acoustic reflected signal.

**8.** The method of claim 6, comprising determining said time of flight based on a distance from the acoustic transmitter to the radiation absorbing region corresponding to said at least one high intensity region, and an estimate of a speed of sound of the emitted focused ultrasound beam.

**9.** The method of claim 6, comprising determining said time of flight based on a detection time of the detected acoustic response to the electromagnetic radiation, said acoustic response corresponding to the high intensity region.

**10.** The method of claim 1, comprising
determining a plurality of high intensity regions in the object image, said high intensity regions corresponding to respective radiation absorbing regions within the object;
generating a plurality of artifact detection images by causing the acoustic transmitter to emit a sequence of focused ultrasound beams being focused on the respective radiation absorbing regions corresponding to different ones of the high intensity regions in the object image, and causing the acoustic receiver to detect an acoustic reflected signal generated by each of the focused ultrasound beams; and
correcting the object image based on the plurality of artifact detection images.

**11.** The method of claim 10, comprising causing the acoustic transmitter to focus the ultrasound beams on radiation absorbing regions corresponding to high intensity regions in the object image for which there is another high intensity region in the object image further away from the acoustic detector.

**12.** The method of claim 1, comprising
generating a plurality of artifact detection images by causing the acoustic transmitter to emit a sequence of focused ultrasound beams based on the same radiation absorbing region corresponding to the high intensity region, wherein the sequence of focused ultrasound beams have different inclination angles with respect to the object, and causing the acoustic receiver to detect a respective acoustic reflected signal generated by each of the focused ultrasound beams; and
correcting the object image based on the plurality of artifact detection images.

**12.** A system for artifact reduction in photoacoustic or thermoacoustic imaging, comprising
a first imaging unit for generating an object image of an object by causing a radiation emitting device to emit an electromagnetic radiation and causing an acoustic receiver to detect an acoustic response;
an image analyzing unit for determining a high intensity region in the object image, said high intensity region corresponding to a radiation absorbing region within the object;
a second imaging unit for generating an artifact detection image by causing an acoustic transmitter to emit a focused ultrasound beam based on a location of the radiation absorbing region and causing an acoustic receiver to detect an acoustic reflected signal; and
an image correction unit for correcting the object image based on the artifact detection image.

**13.** The system of claim 12, further comprising
the radiation emitting device for emitting the electromagnetic radiation;
the acoustic transmitter for emitting the focused ultrasound beam; and
the acoustic receiver or receivers for detecting the acoustic response and the acoustic reflected signal.

**14.** A computer program product comprising instructions causing a processor to perform the steps of:
generating an object image of an object by causing a radiation emitting device to emit an electromagnetic radiation and causing an acoustic receiver to detect an acoustic response;
determining a high intensity region in the object image, said high intensity region corresponding to a radiation absorbing region within the object;
generating an artifact detection image by causing an acoustic transmitter to emit a focused ultrasound beam based on a location of the radiation absorbing region and causing an acoustic receiver to detect an acoustic reflected signal; and
correcting the object image based on the artifact detection image.

## Amended claims

### Amended claims in accordance with Rule 137(2) EPC.

1. A method of reflection artifact reduction in photoacoustic or thermoacoustic imaging by an imaging apparatus, comprising
generating an object image of an object by causing a radiation emitting device to emit an electromagnetic radiation and causing an acoustic receiver to detect an acoustic response;
determining a high intensity region in the object image, said high intensity region corresponding to a radiation absorbing region within the object;
generating an artifact detection image by causing an acoustic transmitter to emit a focused ultrasound beam based on a location of the radiation absorbing region and causing an acoustic receiver to detect an acoustic reflected signal; and
correcting the object image based on the artifact detection image.

2. The method of claim 1, comprising causing the focused ultrasound beam to be focused on said radiation absorbing region.

3. The method of claim 1, comprising causing the focused ultrasound beam to be emitted and the acoustic reflected signal to be detected while controlling the radiation emitting device to refrain from emitting the electromagnetic radiation.

4. The method of claim 1, comprising processing the detected acoustic reflected signal based on a position of the at least one high intensity region in the object image.

5. The method of claim 1, comprising mapping a position in the artifact detection image to a position in the object image based on a position of the high intensity region in the object image.

6. The method of claim 5, wherein the mapping is based on a time of flight of the focused ultrasound beam from the acoustic transmitter to the radiation absorbing region corresponding to said at least one high intensity region.

7. The method of claim 6, wherein the mapping comprises subtracting said time of flight from a detection time of the detected acoustic reflected signal.

8. The method of claim 6, comprising determining said time of flight based on a distance from the acoustic transmitter to the radiation absorbing region corresponding to said at least one high intensity region, and an estimate of a speed of sound of the emitted focused ultrasound beam.

9. The method of claim 6, comprising determining said time of flight based on a detection time of the detected acoustic response to the electromagnetic radiation, said acoustic response corresponding to the high intensity region.

10. The method of claim 1, comprising
determining a plurality of high intensity regions in the object image, said high intensity regions corresponding to respective radiation absorbing regions within the object;
generating a plurality of artifact detection images by causing the acoustic transmitter to emit a sequence of focused ultrasound beams being focused on the respective radiation absorbing regions corresponding to different ones of the high intensity regions in the object image, and causing the acoustic receiver to detect an acoustic reflected signal generated by each of the focused ultrasound beams; and
correcting the object image based on the plurality of artifact detection images.

11. The method of claim 10, comprising causing the acoustic transmitter to focus the ultrasound beams on radiation absorbing regions corresponding to high intensity regions in the object image for which there is another high intensity region in the object image further away from the acoustic detector.

12. The method of claim 1, comprising
generating a plurality of artifact detection images by causing the acoustic transmitter to emit a sequence of focused ultrasound beams based on the same radiation absorbing region corresponding to the high intensity region, wherein the sequence of focused ultrasound beams have different inclination angles with respect to the object, and causing the acoustic receiver to detect a respective acoustic reflected signal generated by each of the focused ultrasound beams; and
correcting the object image based on the plurality of artifact detection images.

13. A system for artifact reduction in photoacoustic or thermoacoustic imaging, comprising
a first imaging unit for generating an object image of an object by causing a radiation emitting device to emit an electromagnetic radiation and causing an acoustic receiver to detect an acoustic response;
an image analyzing unit for determining a high intensity region in the object image, said high intensity region corresponding to a radiation absorbing region within the object;
a second imaging unit for generating an artifact detection image by causing an acoustic transmitter to emit a focused ultrasound beam based on a location of the radiation absorbing region and causing an acoustic receiver to detect an acoustic reflected signal; and
an image correction unit for correcting the object image based on the artifact detection image.

14. The system of claim 13, further comprising
the radiation emitting device for emitting the electromagnetic radiation;
the acoustic transmitter for emitting the focused ultrasound beam; and
the acoustic receiver or receivers for detecting the acoustic response and the acoustic reflected signal.

15. A computer program product comprising instructions causing a processor to perform the steps of:
generating an object image of an object by causing a radiation emitting device to emit an electromagnetic radiation and causing an acoustic receiver to detect an acoustic response;
determining a high intensity region in the object image, said high intensity region corresponding to a radiation absorbing region within the object;
generating an artifact detection image by causing an acoustic transmitter to emit a focused ultrasound beam based on a location of the radiation absorbing region and causing an acoustic receiver to detect an acoustic reflected signal; and
correcting the object image based on the artifact detection image.
